# EUROPEAN PATENT SPECIFICATION

(11) **EP 4 188 294 B1**
(45) Date of publication and mention of the grant of the patent: **18.06.2025**
(21) Application number: 21746163.1
(22) Date of filing: 20.07.2021
(51) Int. Cl.: A61F 13/00, A61F 13/02, A61L 15/64

(54) **BIORESORBABLE DRESSING FOR NEGATIVE-PRESSURE THERAPY**
BIORESORBIERBARER VERBAND FÜR UNTERDRUCKTHERAPIE
PANSEMENT BIORÉSORBABLE POUR THÉRAPIE PAR PRESSION NÉGATIVE

(30) Priority: 30.07.2020 US 202063059032 P
(43) Date of publication of application: 07.06.2023
(73) Proprietor: Solventum Intellectual Properties Company, Maplewood, MN 55144 (US)
(72) Inventor: KHARKAR, Prathamesh Madhav, Dublin Road Athlone, Co. Westmeath, N37XF22 (IE); SCHMIDT, Marisa, Dublin Road Athlone, Co. Westmeath, N37XF22 (IE); ALLEN, Diwi L., Dublin Road Athlone, Co. Westmeath, N37XF22 (IE)
(74) Representative: Simmons & Simmons
(86) International application number: PCT/IB2021/056538
(87) International publication number: WO 2022/023876

(56) References cited:
- WO-A1-2019/089266
- WO-A1-2019/094466

## Description

### TECHNICAL FIELD

The invention set forth in the appended claims relates generally to tissue treatment systems and more particularly, but without limitation, to dressings and systems for tissue treatment with negative pressure**.**

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

### BRIEF SUMMARY

The invention is defied by the appended claims. A selection of optional features of the invention is set out in the dependent claims.

### BRIEF DESCRIPTION OF THE DRAWINGS

Figure 1 is a block diagram of an example embodiment of a therapy system that can provide negative-pressure treatment in accordance with this specification;
Figure 2 is an exploded view of an example of a dressing, illustrating additional details that may be associated with some example embodiments of the therapy system of Figure 1;
Figure 3 is detailed perspective view of a portion of the manifolding layer of Figure 2; and
Figure 4 is a partial cut-away view illustrating additional details that may be associated with some example embodiments of the therapy system of Figure 1.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

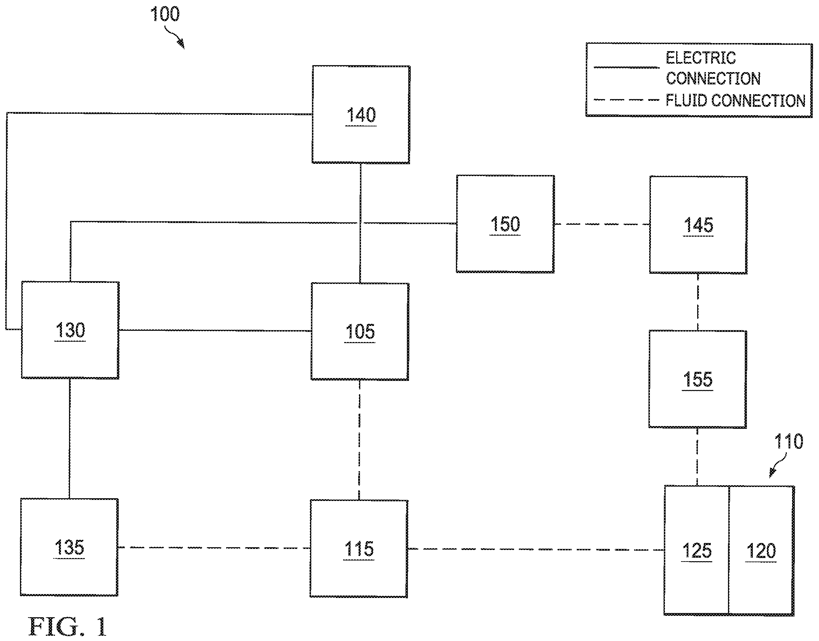

Figure 1 is a block diagram of an example embodiment of a therapy system 100 that can provide negative-pressure therapy to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130 and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may include or may be formed from a manifold. A manifold in this context may comprise a means for collecting or distributing fluid relative to a tissue site under pressure. For example, a manifold may be adapted to receive negative pressure from a source and distribute negative pressure through multiple apertures, which may have the effect of collecting fluid from across a tissue site and drawing the fluid toward the source. In some embodiments, the fluid path may be reversed or a secondary fluid path may be provided to facilitate delivering fluid across a tissue site. In some illustrative embodiments, a manifold may comprise a plurality of pathways, which can be interconnected to improve distribution or collection of fluids. In some illustrative embodiments, a manifold may comprise or be formed from a porous material having interconnected fluid pathways. In some embodiments, a manifold may additionally or alternatively comprise projections that form interconnected fluid pathways. For example, a manifold may be molded to provide surface projections that define interconnected fluid pathways.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or be formed from, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polyamide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inspire 2327 polyurethane films, commercially available from Exopack Advanced Coatings, Wrexham, United Kingdom. In some embodiments, the cover 125 may comprise Inspire 2301 having an MVTR (upright cup technique) of 2600 grams per square meter per twenty-four hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel. The attachment device, for example, an adhesive, may be a layer having substantially the same shape as a periphery of the cover 125. In some embodiments, the adhesive may be continuous or discontinuous. Discontinuities in the adhesive may be provided by apertures or holes (not shown) in the adhesive. The apertures or holes in the adhesive may be formed after application of the adhesive or by coating the adhesive in patterns on a carrier layer, such as, for example, a side of the cover 125. Apertures or holes in the adhesive may also be sized to enhance the MVTR of the dressing 110 in some example embodiments.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The process of reducing pressure may be described illustratively herein as "delivering," "distributing," or "generating" negative pressure, for example. In general, exudate and other fluid flow toward lower pressure along a fluid path. Thus, the term "downstream" typically refers to a location in a fluid path relatively closer to a source of negative pressure or further away from a source of positive pressure. Conversely, the term "upstream" refers to a location in a fluid path relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and such a description should not be construed as limiting.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in the container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, the controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

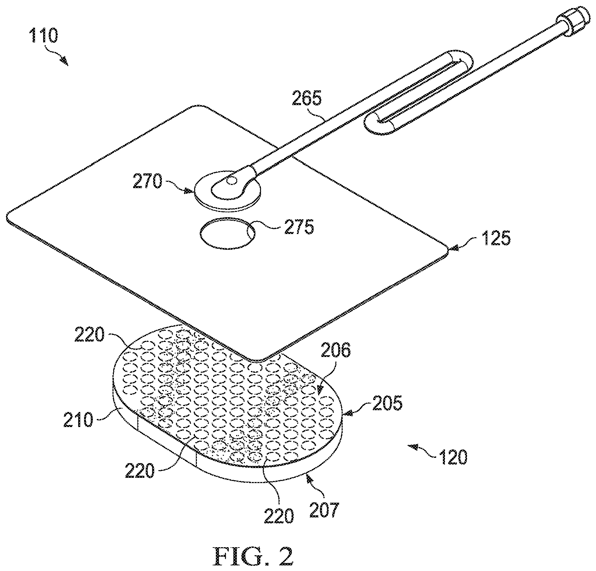

Figure 2 is an exploded view of an example of the dressing 110 of Figure 1, illustrating additional details associated with the tissue interface 120 and cover 125. The example embodiment of Figure 2 illustrates an example where the cover is coupled to a fluid conductor 265 and a dressing interface 270. The fluid conductor 265 may be a flexible tube, which can be fluidly coupled on one end to the dressing interface 270. The dressing interface 270 may be an elbow connector, as shown in the example of Figure 2, which can be placed over an aperture 275 in the cover 125 to provide a fluid path between the fluid conductor 265 and the tissue interface 120.

Figure 2 also illustrates additional details that may be associated with some embodiments in which the tissue interface 120 comprises a manifold layer 205. In some additional embodiments, the tissue interface 120 may include one or more additional layers. In some embodiments, the manifold layer 205 may include a first surface 206, a second surface 207 opposite the first surface 206, and a thickness extending between the first surface 206 and the second surface 207. In some embodiments, the first surface 206, the second surface 207, or both may be generally characterized as planar surfaces, for example, although not necessarily perfectly flat, being generally recognizable as flat or capable of being laid flat. For example, a planar surface may include minor undulations and/or deviations from a single geometric plane. In some embodiments, the first surface 206, the second surface 207, or both may be characterized as continuous. For example, the first surface 206 and/or the second surface 207 may be uninterrupted across its length or width.

In some embodiments, the manifold layer 205 may be configured to provide fluid communication between the first surface 206 and the second surface 207 upon placement of the manifold layer 205 with respect to the tissue site and application of negative pressure to the manifold layer 205. For example, one or more routes of fluid communication between the first surface 206 and the second surface 207 may be formed as a result of placement of the manifold layer 205 with respect to the tissue site and application of negative pressure to the manifold layer 205.

For example, in some embodiments, the manifold layer 205 may comprise a manifolding structure 210 and a plurality of sacrificial zones 220. Each of the plurality of sacrificial zones 220 may be configured to degrade upon placement of the manifold layer 205 with respect to the tissue site and application of negative pressure to the manifold layer 205 so as to allow communication of negative pressure through the manifolding structure 210, for example, between the first surface 206 and the second surface 207.

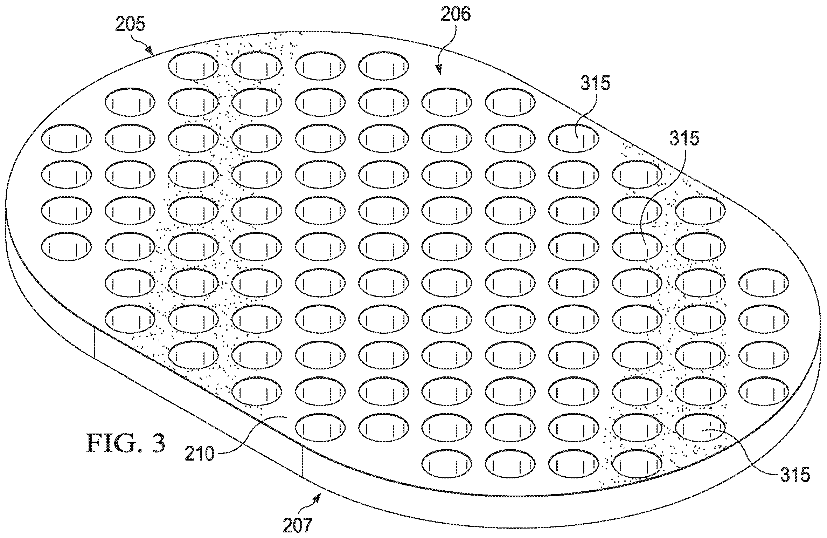

Figure 3 illustrates a detailed perspective view of the manifolding structure 210. In various embodiments, the manifolding structure 210 may comprise a plurality of apertures 315 corresponding to the locations of the plurality of sacrificial zones 220. In various embodiments, the apertures 315 may have any suitable shape in a cross-section generally parallel to the first surface 206 and/or the second surface 207. For example, in various embodiments, one or more of the apertures 315 may be circular, square, rectangular, diamond-shaped, oval, ovoid, irregular, polygonal (for example, hexagonal), or amorphous. The apertures 315 may have a suitable size, for example, a width and/or length from about 4 mm to about 20 mm, or from about 8 mm to about 16 mm, or from about 10 mm to about 15 mm, or from about 12 mm to about 15 mm, or from about 10 mm to about 14 mm, or from about 12 mm to about 14 mm.

In various embodiments, the apertures 315 may be distributed across the manifolding structure 210 in any suitable fashion. For example, as illustrated in Figure 3, the apertures 315 may be distributed across the manifolding structure 210 in a uniform grid of parallel rows and columns. Within each row and/or column, the apertures 315 may be at least substantially equidistantly-spaced with respect to each other. In other embodiments, the apertures 315 may be arranged in another regular pattern or, alternatively, may be disposed randomly.

In some embodiments, the manifolding structure 210, the plurality of sacrificial zones 220, or both may be characterized as biodegradable or as exhibiting biodegradability. As used herein, "biodegradable" and "biodegradability" may refer to a characteristic of a material to at least partially break down and/or exhibit a loss of structural integrity upon exposure to physiological fluids or processes. For example, in some embodiments, the manifolding structure 210, the plurality of sacrificial zones 220, or both may disintegrate, degrade, or dissolve when contacted with an aqueous medium, such as water, blood, or wound exudate from a tissue site. Biodegradability may be a result of a chemical process or condition, a physical process or condition, or combinations thereof.

Additionally or alternatively, in some embodiments, the manifolding structure 210, the plurality of sacrificial zones 220, or both may be characterized as bioresorbable or as exhibiting bioresorbability. As used herein, "bioresorbable" and "bioresorbability" may refer to a characteristic of a material to be broken down into degradation products that may be absorbed at a tissue site so as to be eliminated by the body, for example via metabolism or excretion. In some embodiments, bioresorbability characteristics that may be associated with the manifolding structure 210, the plurality of sacrificial zones 220, or both may be such that at least a portion of the manifolding structure 210 and/or the plurality of sacrificial zones 220 may be eliminated from the tissue site to which it is applied by bioresorption.

In some embodiments, the manifolding structure 210 and/or the plurality of sacrificial zones 220 may be configured to exhibit a desired proportion of disintegration, degradation, or dissolution within a particular time period. For instance, in various embodiments, the manifolding structure 210 and/or the plurality of sacrificial zones 220 may be configured such that at least about 80% by weight, or about 90% by weight, or about 95% by weight, or about 99% by weight, or about 100% by weight of the manifolding structure 210 and/or the plurality of sacrificial zones 220 may be disintegrated, degraded, or dissolved within a desired duration, from contact with a physiological fluid, for example, an aqueous fluid such as blood or wound exudate, at a temperature of about 37° C.

In some embodiments, the manifolding structure 210 may comprise or be formed at least partially from a first composition and the plurality of sacrificial zones 220 may comprise or be formed at least partially from a second composition. In some embodiments, the first composition, the second composition, or both may be characterized as a biodegradable composition. Additionally or alternatively, in some embodiments, the first composition, the second composition, or both may be characterized as a bioresorbable composition. For example, the first composition, the second composition, or both may comprise a biodegradable material, for example, a biodegradable polymer. Additionally or alternatively, the first composition, the second composition, or both may comprise a bioresorbable material, for example, a bioresorbable polymer. In some embodiments, the first composition and the second composition may comprise the same or substantially the components, for example, in varying amounts or concentrations. In other embodiments, the first composition and the second composition may comprise the different components.

For example, in some embodiments, the first composition and the second composition may be configured to exhibit degradation of varying durations. In some embodiments, the first composition may be configured to degrade over a longer duration than the second composition. For example, the first composition may be configured to degrade over a first duration and the second composition may be configured to degrade over a second duration. In various embodiments, the first duration may be at least about 1,000% of the second duration, or the first duration may be at least about 1,500% of the second duration, or the first duration may be at least about 2,000% of the second duration, or the first duration may be at least about 2,500% of the second duration, or the first duration may be at least about 3,000% of the second duration, or the first duration may be at least about 3,500% of the second duration, or the first duration may be at least about 4,000% of the second duration, or the first duration may be at least about 4,500% of the second duration, or the first duration may be at least about 5,000% of the second duration, or the first duration may be at least about 6,000% of the second duration, or the first duration may be at least about 7,000% of the second duration, or the first duration may be at least about 8,000% of the second duration, or the first duration may be at least about 9,000% of the second duration, or the first duration may be at least about 10,000% of the second duration, or the first duration may be at least about 15,000% of the second duration, or the first duration may be at least about 20,000% of the second duration.

In some embodiments, the first composition, the second composition, or both may comprise oxidized cellulose or, in a more particular embodiment, oxidized regenerated cellulose (ORC). The oxidized cellulose may be produced by the oxidation of cellulose, for example with dinitrogen tetroxide. Not intending to be bound by theory, this process may convert primary alcohol groups on the saccharide residues to carboxylic acid group, forming uronic acid residues within the cellulose chain. The oxidation may not proceed with complete selectivity, and as a result, hydroxyl groups on carbons 2 and 3 may be converted to the keto form, for example, comprising ketone units. These ketone units may yield an alkali-labile link, which at pH 7 or higher, may initiate the decomposition of the polymer via formation of a lactone and sugar ring cleavage. As a result, oxidized cellulose may be biodegradable and bioresorbable under physiological conditions.

In some embodiments, the ORC may be prepared by oxidation of a regenerated cellulose, such as rayon. ORC may be manufactured, for example, by the process described in U.S. Patent 3,122,479 to Smith, issued February 24, 1964. ORC is available with varying degrees of oxidation and hence rates of degradation. In some embodiments, the ORC may be in the form of watersoluble low molecular weight fragments obtained by alkali hydrolysis of ORC.

The ORC may be used in a variety of physical forms, including particles, fibers, sheets, sponges, or fabrics. In some embodiments, the ORC is in the form of particles, such as fiber particles or powder particles, for example dispersed in a suitable solid or semisolid topical vehicle. In some embodiments, the first composition, the second composition, or both may comprise ORC fibers, for example, having a volume fraction of at least 80% of the fibers having lengths in the range of from about 20 µm to about 50 mm. In some embodiments, a volume fraction of at least 80% of the fibers have lengths in the range of from about 5 µm to about 1000 µm, or from about 250 µm to about 450 µm. In some embodiments, a volume fraction of at least 80% of the fibers have lengths in the range of from about 25 mm to about 50 mm. Desired size distributions can be achieved, for example, by milling an ORC cloth, followed by sieving the milled powder to remove fibers outside the range. Fabrics may include woven, non-woven and knitted fabrics.

In some embodiments, the oxidized cellulose or ORC may be present in the first composition, the second composition, or both may at any level appropriate. For example, the collagen or other structural protein may be present in the first composition or the second composition at a level of from about 10% to about 80% by weight, or from about 30% to about 60% by weight, or from about 40% to about 50%, or about 45% collagen, by weight of the manifolding structure 210 or the sacrificial zones 220, respectively.

Additionally or alternatively, in some embodiments, the first composition, the second composition, or both may comprise a structural protein. Examples of suitable structural proteins may include, but are not limited to fibronectin, fibrin, laminin, elastin, collagen, gelatins, keratin, and mixtures thereof. For instance, in a particular embodiment, the structural protein comprises, or is, collagen. The collagen may be obtained from any suitable natural source. The collagen may be Type I, Type II, or Type III collagen, or may also be chemically modified collagen, for example, an atelocollagen obtained by removing the immunogenic telopeptides from natural collagen. The collagen may also comprise solubilized collagen or soluble collagen fragments having molecular weights in the range of from about 5,000 to about 100,000 or from about 10,000 to about 50,000, which may be obtained, for example, by pepsin treatment of natural collagen. In various embodiments, the collagen may be obtained from bovine corium that has been rendered largely free of non-collagenous components, for example, free of fat, non-collagenous proteins, polysaccharides and other carbohydrates, as described in U.S. Patent 4,614,794, Easton et al., issued September 30, 1986 and U.S. Patent 4,320,201, Berg et al., issued March 16, 1982.

In some embodiments, the collagen or other structural protein may be present in the first composition, the second composition, or both may at any level appropriate. For example, the collagen or other structural protein may be present in the first composition or the second composition at a level of from about 20% to about 90% by weight, or from about 40% to about 70% by weight, or from about 50% to about 60%, or about 55% collagen, by weight of the manifolding structure 210 or the sacrificial zones 220, respectively.

In some, more particular embodiments, the first composition, the second composition, or both may comprise both ORC and collagen. For example, in some embodiments, the first composition, the second composition, or both comprises ORC at a level of from about 40% to about 50%, or about 45%, and collagen at a level of from about 50% to about 60%, or about 55%, by weight of the manifolding structure 210 or the sacrificial zones 220, respectively.

Additionally, in some embodiments the first composition, the second composition, or both may comprise one or more additional, optional materials. Such optional components may include, for example, preservatives, stabilizing agents, hydrogels and other gelling agents, plasticizers, matrix strengthening materials, dyestuffs, and various active ingredients. In various embodiments, the additional, optional materials may each, when present, be present in a safe and effective amount. As referred to herein, a "safe and effective" amount of a material used herein, refers to an amount that is sufficient to impart a desired effect without undue adverse side effects (such as toxicity, irritation, or likelihood of allergic response), commensurate with a reasonable benefit/risk ratio when used in the manner of this technology. The specific safe and effective amount of a particular material may vary with such factors as the type and quantity of other materials in the composition, the intended use, and the physical condition of the subject of the therapy.

For example, in some embodiments, the first composition, the second composition, or both may comprise an optional gelling agent, examples of which may include, but are not limited to polyurethane gels, modified acrylamide polymers, and hydrophilic polysaccharides. Examples of hydrophilic polysaccharides may include, but are not limited to, alginates, chitosan, chitin, guar gums, pectin, polyethylene glycols, dextrans, starch derivatives, cellulose derivatives (such as hydroxyethyl cellulose, hydroxylpropyl cellulose, and hydroxypropylmethyl cellulose), glycosaminoglycans, galactomannans, chondroitin salts (such as chondroitin sulfate), heparin salts (such as heparin sulfate), hyaluroinic acid and salts thereof, hyaluronates, and mixtures thereof.

For example, in some embodiments, the first composition, the second composition, or both may comprise carboxymethyl cellulose ("CMC"), for example, to modify the rheological, absorbency, or other characteristics of the first composition or the second composition. The CMC may be derived from cellulose and modified such that carboxymethyl groups are bonded to hydroxyl groups in the glucopyranose monomers that make up the cellulose. The CMC may be in salt form, for example, comprising a physiologically acceptable cation, such as sodium (i.e., sodium carboxymethyl cellulose). CMC is commercially available as Walocel^{™} (sold by The Dow Chemical Company) and Cekol^{®} (sold by CP Kelco). When present, the CMC may be present in the first composition or the second composition at any level appropriate to result in the desired characteristics.

In some embodiments, the first composition, the second composition, or both may comprise a strengthening material, which can improve the handling characteristics of the manifold layer 205, the manifolding structure 210, or the sacrificial zones 220. For example, a strengthening material can decrease a substrate's susceptibility to tearing. An example of a suitable strengthening material includes non-gelling cellulose fibers. Such "non-gelling" cellulose fibers may be substantially water-insoluble, and may be produced from cellulose that has not been chemically modified to increase water solubility (as contrasted from carboxymethyl cellulose or other cellulose ethers). Non-gelling cellulose fibers are commercially available as Tencel^{®} fibers (sold by Lenzing AG). Such fibers may be processed from a commercially-available continuous length, by cutting into lengths that are, in some embodiments, from about 0.5 to about 5 cm, or from about 2 to about 3 cm in length. The non-gelling cellulose fibers may be present in the first composition or the second composition at any level appropriate to result in the desired physical characteristics of the manifolding structure 210 or the sacrificial zones 220, respectively.

In some embodiments, the first composition, the second composition, or both may also comprise one or more active ingredients, for example, which may aid in wound healing. Examples of active ingredients include, but are not limited to, non-steroidal anti-inflammatory drugs, acetaminophen, steroids, optional antibiotics and antiseptics, growth factors, peptides, and microRNA. In general, such active ingredients, when present may be present at a level of from about 0.01% to about 10% by weight. As an example, the first composition, the second composition, or both may comprise a growth factor. Examples of suitable growth factors include, but are not limited to, platelet derived growth factor (PDGF), fibroblast growth factor (FGF), and epidermal growth factor (EGF), and mixtures thereof.

Also for example, the first composition, the second composition, or both may comprise an antimicrobial agent, an antiseptic, or both. Examples of antimicrobial agents include, but are not limited to, tetracycline, penicillins, terramycins, erythromycin, bacitracin, neomycin, polymycin B, mupirocin, clindamycin, and combinations thereof. Examples of antiseptics include, but are not limited to silver, polyhexanide (polyhexamethylene biguanide or PHMB), chlorhexidine, povidone iodine, triclosan, sucralfate, quaternary ammonium salts, and combinations thereof. For example, in various embodiments, the first composition, the second composition, or both may comprise silver, which may be in metallic form, in ionic form (e.g., a silver salt), or both. In some embodiments, the first composition, the second composition, or both may comprise a complex of silver and ORC (a "Silver/ORC complex"). For example, a complex of silver and ORC may comprise an intimate mixture at the molecular level, for example, with ionic or covalent bonding between the silver and the ORC. For example, the Silver/ORC complex may comprise a salt formed between the ORC and Ag +, but it may also comprise silver clusters or colloidal silver metal, for example produced by exposure of the complex to light. The complex of an anionic polysaccharide and silver can be made by treating the ORC with a solution of a silver salt. In various embodiments, the silver salt may be the salt of silver with a weak acid. Silver/ORC complexes useful herein, and methods of producing such complexes, are described in U.S. Patent 8,461,410, Cullen et al., issued June 11, 2013. Similar processes are described in U.S. Patent 5,134,229, Saferstein et al., issued July 28, 1992. Alternatively, in other embodiments, an antimicrobial agent or an antiseptic may be absent from the first composition, the second composition, or both.

In some embodiments, such as in embodiments where the first composition, the second composition, or both may comprises silver, the first composition, the second composition, or both may also comprise a dyestuff. The dyestuff may be light-absorbing in the visible region of 400-700 nm. Such dyestuffs may be operable to photochemically trap generated free radicals that could otherwise react with the silver in the present compositions, acting as photochemical desensitisers. In various embodiments, the antioxidant dyestuff may comprise an aniline dye, an acridine dye, a thionine dye, a bis-naphthalene dye, a thiazine dye, an azo dye, an anthraquinone dye, and combinations thereof. For example, the antioxidant dyestuff may comprise gentian violet, aniline blue, methylene blue, crystal-violet, acriflavine, 9-aminoacridine, acridine yellow, acridine orange, proflavin, quinacrine, brilliant green, trypan blue, trypan red, malachite green, azacrine, methyl violet, methyl orange, methyl yellow, ethyl violet, acid orange, acid yellow, acid blue, acid red, thioflavin, alphazurine, indigo blue, methylene green, and combinations thereof. If present, the dyestuff may be present in the first composition, the second composition, or both at a level of about 0.05% to about 5%, or about 0.2% to about 2% by weight of the manifolding structure 210 or the sacrificial zones 220, respectively.

In some embodiments, the first composition, the second composition, or both may be configured to exhibit or impart one or more beneficial effects to the manifolding structure 210 or the sacrificial zones 220, respectively, when the dressing 110 is deployed in a physiological environment, for example, with respect to a tissue site. For example, the first composition, the second composition, or both may be configured to exhibit or impart protease-inhibiting activity, antimicrobial activity, or combinations thereof. For example, in some embodiments, the manifolding structure 210, the sacrificial zones 220, or both may be configured to modulate protease activity. For example, contact with a wound fluid, such as wound exudate, may cause the manifolding structure 210, the sacrificial zones 220, or both to break down into products that may have the effect of modulating protease activity, which may include inhibiting protease activity. For example, the disintegration, degradation, and/or dissolution products of collagen and/or ORC may be effective to inhibit the activity of destructive enzymes such as neutrophil elastase and matrix metalloproteinase (MMP) at a tissue site. In various embodiments, the manifolding structure 210, the sacrificial zones 220, or both may be effective to inhibit protease activity such that protease activity is decreased to less than about 75% of the protease activity than would be present if uninhibited, or to less than about 50%, or to less than about 40%, or to less than about 30% to less than about 20% of the protease activity that would be present if uninhibited.

In some embodiments, the first composition and the second composition may be characterized with respect to solids-content. In some embodiments, the first composition may be characterized as having a greater solids-content than the second composition. For example, the first composition may be characterized as having a first solids-content and the second composition may be characterized as having a second solids-content. In some embodiments, the first solids-content may be from about 0.5% to about 10% by volume, or from about from about 1.0% to about 7.5%, or from about 1.25% to about 5.0%, or from about 1.5% to about 5.0%, or of about 2.0%. Additionally or alternatively, in some embodiments, the second solids-content may be from about 0.01% to about 1.0% by volume, or from about from about 0.025% to about 0.8%, or from about 0.05% to about 0.6%, or from about 0.075% to about 0.2%, or of about 0.1%. For example, in various embodiments, the first solids-content may be at least about 1,000% of the second solids-content, or the first solids-content may be at least about 1,500% of the second solids-content, or the first solids-content may be at least about 2,000% of the second solids-content, or the first solids-content may be at least about 2,500% of the second solids-content, or the first solids-content may be at least about 3,000% of the second solids-content, or the first solids-content may be at least about 3,500% of the second solids-content, or the first density may be at least about 4,000% of the second solids-content, or the first solids-content may be at least about 4,500% of the second solids-content, or the first solids-content may be at least about 5,000% of the second solids-content.

Additionally or alternatively, in some embodiments, the first composition and the second composition may be characterized as exhibiting varying densities. In some embodiments, the first composition may be characterized as having a greater density than the second composition. For example, the first composition may be characterized as having a first density and the second composition may be characterized as having a second density. In various embodiments, the first density may be at least about 1,000% of the second density, or the first density may be at least about 1,500% of the second density, or the first density may be at least about 2,000% of the second density, or the first density may be at least about 2,500% of the second density, or the first density may be at least about 3,000% of the second density, or the first density may be at least about 3,500% of the second density, or the first density may be at least about 4,000% of the second density, or the first density may be at least about 4,500% of the second density, or the first density may be at least about 5,000% of the second density, or the first density may be at least about 6,000% of the second density, or the first density may be at least about 7,000% of the second density, or the first density may be at least about 8,000% of the second density, or the first density may be at least about 9,000% of the second density, or the first density may be at least about 10,000% of the second density, or the first density may be at least about 15,000% of the second density, or the first density may be at least about 20,000% of the second density.

In some embodiments, the manifold layer 205 may be formed by a process that comprises preparing a first solution. The first solution may comprise a biodegradable material, such as a biodegradable polymer, and/or a bioresorbable material, such as a bioresorbable polymer. For example, in some embodiments, the first solution may comprise collagen and ORC. The first solution may also comprise one or more preservatives, stabilizing agents, hydrogels and other gelling agents, plasticizers, matrix strengthening materials, dyestuffs, and various active ingredients, for example, silver. In some embodiments, the first solution may have a solids-content from about 0.5% to about 10% by volume of the first solution, or from about from about 1.0% to about 7.5%, or from about 1.25% to about 5.0%, or from about 1.5% to about 5.0%, or of about 2.0% by volume of the first solution. Additionally, the first solution may include a carrier fluid, for example, an aqueous fluid such as purified, deionized, or distilled water.

The process for forming the manifold layer 205 may also comprise forming the manifolding structure 210. In some embodiments, the manifolding structure 210 may be formed by freeze-drying (e.g., lyophilizing) the first solution. For example, the first solution may be disposed in a mold or form which may have the desired shape for the manifolding structure 210. For example, in some embodiments, the mold or form may include a plurality of projections configured to form the apertures 315 within the manifolding structure 210. Alternatively, in some embodiments, the first solution may be freeze-dried in any suitable form, for example, a sheet, and later trimmed or sized to form the manifolding structure 210. Also, in some embodiments the apertures 315 may be formed within the manifolding structure 210 after freeze-drying, such as by perforating the freeze-dried structure.

In some embodiments, the process for forming the manifold layer 205 may also comprise preparing a second solution. Similar to the first solution, the second solution may comprise a biodegradable material, such as a biodegradable polymer, and/or a bioresorbable material, such as a bioresorbable polymer. For example, in some embodiments, the second solution may comprise collagen and ORC. Also, the second solution may also comprise one or more preservatives, stabilizing agents, hydrogels and other gelling agents, plasticizers, matrix strengthening materials, dyestuffs, and various active ingredients, for example, silver. In some embodiments, the second solution may have a solids-content from about 0.01% to about 1.0% by volume, or from about from about 0.025% to about 0.8%, or from about 0.05% to about 0.6%, or from about 0.075% to about 0.2%, or of about 0.1% by volume of the second solution. Additionally, and also like the first solution, the second solution may include a carrier fluid, for example, an aqueous fluid such as purified, deionized, or distilled water.

The process for forming the manifold layer 205 may also comprise forming the sacrificial zones 220. In some embodiments, the sacrificial zones 220 may be formed by freeze-drying (e.g., lyophilizing) the second solution. For example, the second solution may be disposed within the apertures 315 of the manifolding structure 210 and freeze-dried while in place.

In various embodiments, the first composition, the second composition, or both may be essentially free of water, for example, as a result of the freeze-drying (e.g., lyophilization). For example, in some embodiments, the first composition, the second composition, or both may comprise 10% or less, 8% or less, or 5% or less, of water.

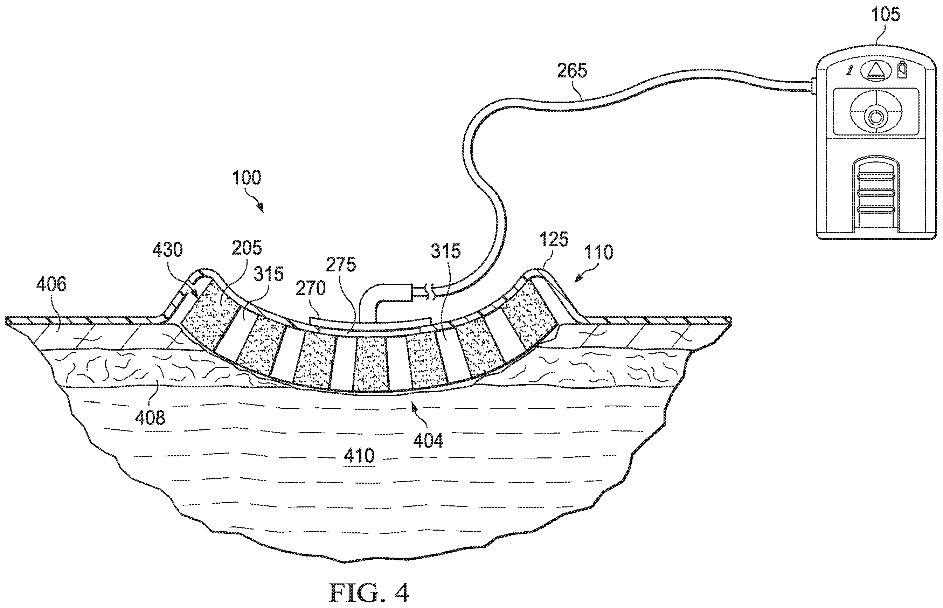

A system comprising the dressing 110 may be advantageously employed to provide negative-pressure therapy to a user. For example, Figure 4 depicts an embodiment of the therapy system 100 positioned for the treatment of a tissue site 404. The tissue site 404 may extend through or otherwise involve peripheral tissue, for example, an epidermis 406, a dermis 408, and a subcutaneous tissue 410. Additionally or alternatively, in some embodiments, the tissue site 404 may include a surface portion that predominantly resides on the surface of the epidermis 406, such as, for example, an incision. The tissue site 404 may have a depth extending beneath the surface of the peripheral tissue, for example, to or into the epidermis 406 or dermis. The system therapy 100 may provide therapy to, for example, the epidermis 406, the dermis 408, and the subcutaneous tissue 410, regardless of the positioning of the therapy system 100 or the type of tissue site. The therapy system 100 may also be utilized without limitation at other tissue sites.

The dressing 110 may be positioned with respect to the tissue site 404 such that the manifold layer 205 is at or proximate to the tissue site 404. When initially positioned with respect to the tissue site 404, the first surface 206 and/or the second surface 207 of the manifold layer 205 may each be continuous or substantially continuous. For example, when initially positioned with respect to the tissue site 404, the sacrificial zones 220 may be intact, such that the apertures 315 do not allow for fluid communication between the first surface 206 and the second surface 207 of the manifold layer 205.

The dressing 110 may also be positioned with respect to the tissue site 404 such that the cover 125 may cover manifold layer 205 and the tissue site 404 to provide a fluid seal between the tissue site 404 and the cover 125 of the dressing 110, for example, to yield a sealed space 430. Further, the cover 125 may cover other tissue, such as a portion of the epidermis 406, surrounding the tissue site 404 to provide the fluid seal between the cover 125 and the tissue site 404. In some embodiments, a portion of the periphery of the cover 125 may extend beyond the manifold layer 205 and into direct contact with tissue surrounding the tissue site 404.

With the dressing 110 positioned and secured with respect to the tissue site 404, a conduit may be coupled between the negative-pressure source 105 and the dressing 110 and the negative-pressure source 105 may be operated to provide negative-pressure therapy to the tissue site 404, for example, via the sealed space 430 and/or the dressing 110.

In some embodiments, upon placement of the manifold layer 205 with respect to the tissue site 404, the manifold layer 205 may be exposed to various physiological fluids, for example, wound exudate. In some embodiments, exposure to physiological fluids may cause the manifolding structure 210, the sacrificial zones 220, or both to be at least partially degraded. Not intending to be bound by theory, the proportion of and/or degree to which the sacrificial zones 220 are degraded may be greater than the proportion of and/or degree to which the manifolding structure 210 is degraded. Additionally or alternatively, the application of the negative pressure to the sealed space 430 and/or the dressing 110 may be effective to further degrade the sacrificial zones 220, to cause a loss of structural integrity with respect to the sacrificial zones 220, to cause the sacrificial zones 220 to rupture or collapse, or combinations thereof. For example, the application of the manifold layer 205 to the tissue site 404 may cause the second composition forming the sacrificial zones 220 to be at least partially degraded, for example, softened or weakened, and the application of negative pressure to the manifold layer 205 may cause the second composition forming the sacrificial zones 220 to be removed from the apertures 315. As such, upon application of the manifold layer 205 with respect to the tissue site and application of negative pressure to the sealed space may cause the sacrificial zones 220 to degrade such that the manifold layer 205 will communicates negative pressure between the first surface 206 and the second surface 207 via the apertures 315.

In some embodiments, as the therapy progresses, the manifolding structure 210 may be biodegraded and/or bioresorbed. In some embodiments, the degradation products of the manifolding structure 210 may be effective to inhibit the activity of destructive enzymes such as neutrophil elastase and matrix metalloproteinase (MMP) at the tissue site 404 while the manifolding structure 210 continues to be effective to manifold negative pressure. In various embodiments, the duration over which the manifolding structure is degraded may be tailored to meet the needs of a particular therapy.

In some embodiments, the dressing 110 may be advantageously employed in the provision of negative-pressure therapy. For example, the presence of the sacrificial zones 220 within the manifold layer 205 may allow for the manifolding of negative pressure via the manifold layer 205 while also improving the ease with which the manifold layer 205 can be positioned with respect to the tissue site 404 and/or may allow for the manifold layer 205 to be repositioned after an initial attempt at placement. For example, the presence of the sacrificial zones 220 may improve the structural or handling characteristics of the manifold layer 205 in comparison to a degradable and/or bioresorbable layer comprising apertures but no sacrificial material to, at least initially, fill the apertures.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or separated in various configurations for purposes of sale, manufacture, assembly, or use. In some configurations, various components, for example, the dressing 110 or the container 115, may be separated from other components for manufacture or sale. In other example configurations, the controller 130 may also be manufactured, configured, assembled, or sold independently of other components.

## Claims

1. A dressing for treating a tissue site with negative pressure, the dressing comprising:
a bioresorbable manifold layer (205) comprising:
a first surface (206),
a second surface (207) opposite the first surface (206),
a thickness extending between the first surface (206) and the second surface (207), and
a plurality of sacrificial zones (220) configured to degrade upon placement of the bioresorbable manifold layer (205) with respect to the tissue site and application of negative pressure to the bioresorbable manifold layer (205) so as to allow communication of negative pressure between the first surface (206) and the second surface (207);
a bioresorbable manifolding structure (210) formed from a first bioresorbable composition and comprising a plurality of apertures (315) extending between the first surface (206) and the second surface (207) and corresponding to each of the plurality of sacrificial zones (220); and
a second bioresorbable composition disposed within each of the plurality of apertures (315) to form the sacrificial zones (220).

2. The dressing of claim 1, wherein at least one of the first surface (206) and the second surface (207) is a continuous surface.

3. The dressing of one of claims 1-2, wherein the first surface (206) and the second surface 9207) are continuous surfaces.

4. The dressing of claim 1, wherein the first bioresorbable composition is configured to degrade over a first duration and the second bioresorbable composition is configured to degrade over a second duration, wherein the first duration is at least about 1000% of the second duration.

5. The dressing of claim 4, wherein the first duration is at least about 2500% of the second duration.

6. The dressing of one of claims 4-5, wherein the first duration is at least about 5000% of the second duration.

7. The dressing of one of claims 1-6, wherein the first bioresorbable composition exhibits a first density and the second bioresorbable composition exhibits a second density, wherein the first density is at least about 1000% of the second density.

8. The dressing of claim 7, wherein the first density is at least about 1500% of the second density, or at least about 2000% of the second density.

9. The dressing of one of claims 1-8, wherein the first and/or second bioresorbable composition comprises collagen and oxidized regenerated cellulose.

10. The dressing of one of claims 1-9, wherein each of the first bioresorbable composition and the second bioresorbable composition comprises from about 50% to about 60% collagen by weight and from about 40% to about 50% oxidized regenerated cellulose by weight.

11. The dressing of one of claims 1-10, wherein each of the first bioresorbable composition and the second bioresorbable composition comprises an antimicrobial agent.

12. The dressing of one of claims 1-11, wherein the bioresorbable manifold layer is formed from a process comprising:
preparing a first solution;
forming the bioresorbable manifolding structure (210) by freeze-drying the first solution;
preparing a second solution; and
freeze-drying the second solution while disposed within the plurality of apertures (315).

13. A system for treating a tissue site with negative pressure, the system comprising:
a dressing according to any preceding claim; and
a negative-pressure source (105) configured to be fluidly coupled to the dressing.

## Patentansprüche

1. Ein Verband zum Behandeln einer Gewebestelle mit Unterdruck, der Verband aufweisend:
eine bioresorbierbare Verteilerschicht (205), aufweisend:
eine erste Oberfläche (206),
eine zweite Oberfläche (207) gegenüber der ersten Oberfläche (206),
eine Dicke, die sich zwischen der ersten Oberfläche (206) und der zweiten Oberfläche (207) erstreckt, und
eine Vielzahl von Opferzonen (220), die konfiguriert sind, um sich bei einer Platzierung der bioresorbierbaren Verteilerschicht (205) hinsichtlich der Gewebestelle und bei einer Anwendung von Unterdruck auf die bioresorbierbare Verteilerschicht (205) zu zersetzen, um Austausch von Unterdruck zwischen der ersten Oberfläche (206) und der zweiten Oberfläche (207) zu ermöglichen;
eine bioresorbierbare Verteilerstruktur (210), die aus einer ersten bioresorbierbaren Zusammensetzung ausgebildet ist und aufweisend eine Vielzahl von Öffnungen (315), die sich zwischen der ersten Oberfläche (206) und der zweiten Oberfläche (207) erstrecken und jeder der Vielzahl von Opferzonen (220) entsprechen; und
eine zweite bioresorbierbare Zusammensetzung, die innerhalb jeder der Vielzahl von Öffnungen (315) angeordnet ist, um die Opferzonen (220) auszubilden.

2. Der Verband nach Anspruch 1, wobei mindestens eine der ersten Oberfläche (206) und der zweiten Oberfläche (207) eine fortlaufende Oberfläche ist.

3. Der Verband nach einem der Ansprüche 1 bis 2, wobei die erste Oberfläche (206) und die zweite Oberfläche 9207) fortlaufende Oberflächen sind.

4. Der Verband nach Anspruch 1, wobei die erste bioresorbierbare Zusammensetzung konfiguriert ist, um sich über eine erste Dauer zu zersetzen, und die zweite bioresorbierbare Zusammensetzung konfiguriert ist, um sich über eine zweite Dauer zu zersetzen, wobei die erste Dauer mindestens etwa 1000 % der zweiten Dauer beträgt.

5. Der Verband nach Anspruch 4, wobei die erste Dauer mindestens etwa 2500 % der zweiten Dauer beträgt.

6. Der Verband nach einem der Ansprüche 4 bis 5, wobei die erste Dauer mindestens etwa 5000 % der zweiten Dauer beträgt.

7. Der Verband nach einem der Ansprüche 1 bis 6, wobei die erste bioresorbierbare Zusammensetzung eine erste Dichte vorweist und die zweite bioresorbierbare Zusammensetzung eine zweite Dichte vorweist, wobei die erste Dichte mindestens etwa 1000 % der zweiten Dichte beträgt.

8. Der Verband nach Anspruch 7, wobei die erste Dichte mindestens etwa 1500 % der zweiten Dichte oder mindestens etwa 2000 % der zweiten Dichte beträgt.

9. Der Verband nach einem der Ansprüche 1 bis 8, wobei die erste und/oder die zweite bioresorbierbare Zusammensetzung Kollagen und oxidierte regenerierte Zellulose aufweist.

10. Der Verband nach einem der Ansprüche 1 bis 9, wobei jede der ersten bioresorbierbare Zusammensetzung und der zweiten bioresorbierbaren Zusammensetzung von etwa 50 bis etwa 60 Gew.-% Kollagen und von etwa 40 bis etwa 50 Gew.-% oxidierte regenerierte Zellulose aufweist.

11. Der Verband nach einem der Ansprüche 1 bis 10, wobei jede der ersten bioresorbierbaren Zusammensetzung und der zweiten bioresorbierbaren Zusammensetzung ein antimikrobielles Mittel aufweist.

12. Der Verband nach einem der Ansprüche 1 bis 11, wobei die bioresorbierbare Verteilerschicht aus einen Verfahren ausgebildet wird, aufweisend:
Herstellen einer ersten Lösung;
Ausbilden der bioresorbierbaren Verteilerstruktur (210) durch Gefriertrocknen der ersten Lösung;
Herstellen einer zweiten Lösung; und
Gefriertrocknen der zweiten Lösung, während sie in der Vielzahl von Öffnungen (315) angeordnet ist.

13. System zum Behandeln einer Gewebestelle mit Unterdruck, das System aufweisend:
einen Verband nach einem der vorstehenden Ansprüche; und
eine Unterdruckquelle (105), die konfiguriert ist, um mit dem Verband fluidisch gekoppelt zu werden.

## Revendications

1. Pansement permettant de traiter un site tissulaire par pression négative, le pansement comprenant :
une couche de collecteur biorésorbable (205) comprenant :
une première surface (206),
une seconde surface (207) opposée à la première surface (206) ;
une épaisseur s'étendant entre la première surface (206) et la seconde surface (207), et
une pluralité de zones sacrificielles (220) conçues pour se dégrader lors de la mise en place de la couche de collecteur biorésorbable (205) par rapport au site tissulaire et de l'application d'une pression négative à la couche de collecteur biorésorbable (205) de manière à permettre la communication d'une pression négative entre la première surface (206) et la seconde surface (207) ;
une structure de collecteur biorésorbable (210) formée à partir d'une première composition biorésorbable et comprenant une pluralité d'ouvertures (315) s'étendant entre la première surface (206) et la seconde surface (207) et correspondant à chacune de la pluralité de zones sacrificielles (220) ; et
une seconde composition biorésorbable disposée dans chacune des ouvertures (315) pour former les zones sacrificielles (220).

2. Pansement selon la revendication 1, dans lequel au moins l'une de la première surface (206) et de la seconde surface (207) est une surface continue.

3. Pansement selon l'une quelconque des revendications 1 à 2, dans lequel la première surface (206) et la seconde surface (9207) sont des surfaces continues.

4. Pansement selon la revendication 1, dans lequel la première composition biorésorbable est conçue pour se dégrader sur une première durée et la seconde composition biorésorbable est conçue pour se dégrader sur une seconde durée, dans lequel la première durée est au moins égale à environ 1000 % de la seconde durée.

5. Pansement selon la revendication 4, dans lequel la première durée est au moins égale à environ 2500 % de la seconde durée.

6. Pansement selon l'une quelconque des revendications 4 à 5, dans lequel la première durée est au moins égale à environ 5000 % de la seconde durée.

7. Pansement selon l'une quelconque des revendications 1 à 6, dans lequel la première composition biorésorbable présente une première densité et la seconde composition biorésorbable présente une seconde densité, dans lequel la première densité est au moins égale à environ 1000 % de la seconde densité.

8. Pansement selon la revendication 7, dans lequel la première densité est au moins égale à environ 1500 % de la seconde densité, ou au moins environ 2000 % de la seconde densité.

9. Pansement selon l'une quelconque des revendications 1 à 8, dans lequel la première et/ou la seconde composition biorésorbable comprend du collagène et de la cellulose régénérée oxydée.

10. Pansement selon l'une quelconque des revendications 1 à 9, dans lequel la première composition biorésorbable et la seconde composition biorésorbable comprennent chacune environ 50 % à 60 % de collagène en poids et environ 40 % à 50 % de cellulose régénérée oxydée en poids.

11. Pansement selon l'une quelconque des revendications 1 à 10, dans lequel la première composition biorésorbable et la seconde composition biorésorbable comprennent chacune un agent antimicrobien.

12. Pansement selon l'une quelconque des revendications 1 à 11, dans lequel la couche de collecteur biorésorbable est formée à partir d'un processus comprenant :
la préparation d'une première solution ;
la formation de la structure manifold biorésorbable (210) en lyophilisant la première solution ;
la préparation d'une seconde solution ; et
la lyophilisation de la seconde solution lorsqu'elle est disposée à l'intérieur de la pluralité d'ouvertures (315).

13. Système permettant de traiter un site tissulaire par pression négative, le système comprenant :
un pansement selon l'une quelconque revendication précédente ; et
une source de pression négative (105) conçue pour être accouplée fluidiquement au pansement.
